Europäisches Patentamt

European Patent Office

Office européen des brevets

(19)

(11) Publication number: **0 112 077**
**A1**

(12)  **EUROPEAN PATENT APPLICATION**

(21) Application number: **83307165.7**

(22) Date of filing: **23.11.83**

(51) Int. Cl.³: **C 12 Q 1/00**
**C 12 Q 1/04**

(30) Priority: **14.12.82 GB 8235550**

(43) Date of publication of application:
**27.06.84 Bulletin 84/26**

(84) Designated Contracting States:
**AT BE CH DE FR GB IT LI LU NL SE**

(71) Applicant: **OXOID LIMITED**
**Wade Road**
**Basingstoke Hampshire RG24 0PW(GB)**

(72) Inventor: **Swaine, Derwent**
**24, Edgar Road**
**Winchester Hampshire(GB)**

(74) Representative: **Pennant, Pyers et al,**
**Stevens, Hewlett & Perkins 5 Quality Court Chancery**
**Lane**
**London, WC2A 1HZ(GB)**

(54) Diagnostic test probe.

(57) A diagnostic probe, for testing for the presence in a sample of an enzyme, comprises a handle and a tip, which tip is shaped to be applied to the sample and carries a reagent to react with the enzyme and thereby generate a colour signal. The probe may be a rod or stick of rolled paper or open-pore plastics material. The sample may be a bacterial colony growing on a nutrient gel. The test takes seconds, or at most minutes, and does not involve incubation.

EP 0 112 077 A1

- 1 -

DIAGNOSTIC TEST PROBE

This invention relates to diagnostic test probes for testing for the presence of enzymes.

In microbiology laboratories, a common diagnostic technique involves providing a chosen chemical reagent on a microscope slide, transferring a sample believed to contain a particular micro-organism to the slide, incubating the sample-reagent mixture, and observing whether a chemical reaction takes place. A platinum loop may be used to transfer the sample to the slide and there to mix it with the reagent.

Though widely used, this diagnostic technique is somewhat tedious, and also potentially hazardous when pathogenic bacteria are involved. A simpler and safer technique would be welcomed by microbiology laboratories.

British Patent Specifications 779514, 1082459, 1365001 and 1406180 describe probes for bacterial testing. These carry a nutrient medium and possibly also a colour change reagent. They are designed to be contacted with a sample possibly containing bacteria and then incubated until the bacteria can be observed or identified. Such a test takes hours, or more usually days. By contrast, the probe of the present invention is useful where bacteria have already been incubated, and the enzyme identification test takes only seconds or minutes.

British Patent Specifications 1246080 and 1361577 describe a test for nitrite involving the use of a diagnostic strip carrying various reagents. A bacterial culture is suspended in a tube of saline, and the strip immersed in the tube. The use of a tube containing saline is tedious when many tests have to be carried out, and the saline dilutes the sample thus rendering the test less sensitive.    Furthermore, the test is not for an enzyme, but for a specific inorganic product of bacterial metabolism.

Prior tests for enzymes have used liquid reagents that have either been stored in bulk or have been freshly prepared.    One technique involves applying a drop of the reagent to a bacterial colony growing on a nutrient gel in order to observe a possible colour change;    but that entails destruction of the colony. In another technique, a piece of paper is impregnated with the reagent and a sample of the colony applied to the impregnated area;    this technique is messy and can result, by scratching the paper, in formation of an aerosol of possibly pathogenic bacteria.    A third technique is to suspend a loop of the colony in a tube containing nutrient medium and a colour reagent;    but this is slow and tedious.    Yet another technique involves impregnating a paper disc with the reagent and pressing the disc briefly onto the colony growing on a nutrient gel;    but this involves destroying the colony; also, the reagent unavoidably contacts the nutrient medium, and this is quite often found to affect the diagnostic test.

In one aspect, the present invention provides a diagnostic test probe, for testing for the presence in a sample of an enzyme, the probe comprising a handle and a tip, which tip is shaped to be applied to the sample and carries a reagent to react with the enzyme and thereby generate a signal indicating the presence in the sample of the enzyme.

In another aspect, the invention provides a method of testing for the presence in a sample of an enzyme by the use of a diagnostic test probe as hereinbefore defined, which method comprises applying the reagent-carrying tip of the probe to the sample, permitting reaction to take place between the reagent and the enzyme, and observing the signal indicating the presence in the sample of the enzyme.

One example of a diagnostic test probe according to the invention is a stick of a porous inert material, one end of which has been dipped in a solution of a desired reagent so as to deposit some of the reagent thereon (or therein).

The probe has a handle, whose purpose is to permit manipulation of the probe, and whose shape and size is not critical. When the probe is a stick, one end of the stick may constitute the handle and the other end the tip.

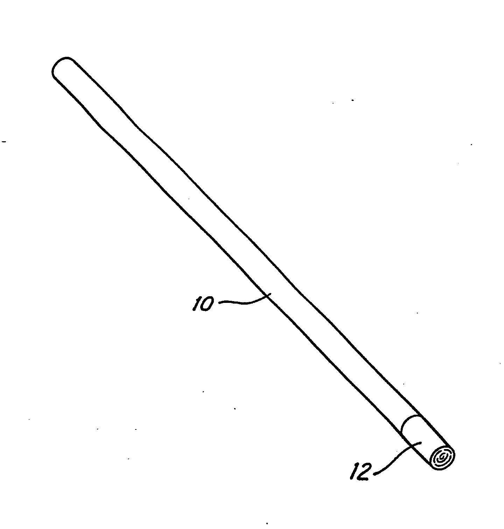

Reference is directed to the accompanying drawing, which is a perspective view of a diagnostic test probe according to the invention comprising a stick of rolled paper 60 mm long, and including a handle portion 10 and a tip 12 which has been dipped in reagent.

The tip of the probe should preferably be large enough for any signal generated thereon to be clearly and quickly observed; and small enough to be accurately located and applied to a small sample, for example a small bacterial colony on an agar gel plate. The tip will generally have a diameter of a few millimetres, for example, from 0.5 to 10 mm, although it need not of course be of circular cross-section.

It is preferred, though not absolutely necessary, that the probe should be self supporting and substantially rigid in use. This preference is particularly marked when the tip of the probe is intended to be pressed against a gel or solid sample, for which purpose a flimsy probe such as a sheet of paper would hardly be suitable.

The sample is preferably a bacterial colony, preferably a colony which has been grown on a nutrient gel such as agar gel. In such a colony, any enzymes produced by the bacteria are present in concentrated form and readily detectable. The test probe of this invention can be applied to the colony without destroying the whole of it, and without touching the nutrient gel on which it is growing. Thus, the diagnostic test is not affected by the nutrient gel; and if the test result is interesting, the colony remains for further identification.

The reagent on (or in) the tip of the probe is chosen to react with the enzyme suspected to be present in the sample and so generate an observable signal. A convenient signal is a colour change. Many colour change reactions are known and used in microbiological diagnosis; see for example "Biochemical Tests for Identification of Medical Bacteria" by J.F. McFadden, published by Williams and Wilkins, U.S.A. When the presence of the enzyme is to be signalled by a colour change, the tip of the probe is preferably of a colour, e.g. white, such that the change is readily observed.

Such colour changes occur rapidly, e.g. in seconds or at most minutes. Incubation of bacteria in contact with the probe is not necessary, and hence the reagent does not include a bacterial nutrient medium.

The enzyme may be caused to react in various ways. For example, reaction may be with reagent chosen to yield directly a coloured product. Alternatively, the reagent may be of a mixture of substances, the reaction of one of which with the enzyme may generate a colour change in another. Thus in Example 3 below, the reaction of urease on urea generates ammonia which raises the pH and turns phenol red from yellow to red.

The tip of the probe is preferably, though not necessarily, porous or adsorbent. Pores of capillary size enable a large amount of reagent, or later of sample, to be taken up by a small tip. But it is also necessary, unless the tip is transparent or transluscent to the emitted signal, that a substantial proportion of the reagent, and later of the sample, be present on the surface of the tip rather than within it.  Taking into account the hydrophilic/hydrophobic nature and the wetting properties of the material of which the tip of the probe is made, it is possible by routine trial and error to find the optimum porosity for the purpose.

The tip should be inert to the reagent and to any chemical that may be present in the sample.  Suitable materials include plastics materials, particularly ones with open-cell pores, cellulose-based products such as rolled paper or card, and matted fibre products such as felt.

The diagnostic test probes with which the invention is concerned are envisaged as cheap products to be disposed of after a single use.  Suitable cheap products are sticks of rolled paper such as are used for lollipop sticks, and rods of porous plastics materials such as polyethylene or polypropylene.  Rods of porous polyethylene and polypropylene with average pore sizes in the range of 10 to 250 microns are available commercially.  A desired reagent may be applied to the ends of these sticks or rods by manual dipping or by more sophisticated methods.  The probes are preferably supplied in sterile form, and may be individually wrapped or packaged.

The following Examples illustrate the invention. In each of Examples 1 to 3, the probe was a rod of porous polyethylene about 7 cm long and 4 mm diameter. In Examples 4 and 5, sticks of rolled paper (lollipop sticks) were used.

## EXAMPLE 1

A probe was formed by dipping one end of one of these rods in a solution of 1 mg Nitrocefin in 2 ml ethanol and allowed to dry.  The tip of the probe was applied to a colony of a penicillin-resistant bacterial strain growing on agar gel.  Beta-lactamase, formed by the bacteria, reacted with the Nitrocefin and caused the tip of the probe to turn from pale yellow to bright red. This change took a few seconds. The probe was thus suitable for testing for the presence of beta-lactamase in a sample.

Nitrocefin is a chromogenic cephalosporin developed by Glaxo Research Limited, (coded 87/312; 3-(2,4 dinitrostyryl-(6R, 7R)-7B(2-thienylacetamido)-ceph-3-em-4-carboxylic acid, E-isomer).

## EXAMPLE 2

A probe was formed by dipping one end of a rod into a water-based reagent comprising m-dimethyl-p-phenylenediamine, oxalate, ascorbic acid and alpha-naphthol. The resulting probe was suitable for testing for cytochrome oxidase, in the presence of which the tip turned from colourless to dark red/black.

## EXAMPLE 3

A probe was formed by dipping one end of a rod into an aqueous medium containing yeast extract, phosphate buffer, urea and phenol red. The resulting probe was suitable for testing for urease, in the presence of which the tip turned from yellow to red.

## EXAMPLE 4

A probe was formed by dipping one end of a stick into a water-based reagent comprising L-Phenylalanine and ferric chloride. The resulting probe was suitable for testing for phenylalanine deaminase enzyme, in the presence of which the tip turned from colourless to green.

## EXAMPLE 5

A probe was formed by dipping one end of a stick into a water-based reagent comprising Ornithine, 0.004 m potassium acid phthalate buffer, pyridoxal phosphate and bromophenol red. The resulting probe was suitable for testing for Ornithine decarboxylase, in presence of which the tip turned from yellow to rose-red.

## C L A I M S

1.    A diagnostic test probe, for testing for the presence in a sample of an enzyme, the probe comprising a handle and a tip, which tip is shaped to be applied to the sample and carries a reagent to react with the enzyme and thereby generate a signal indicating the presence in the sample of the enzyme.

2.    A diagnostic test probe as claimed in claim 1, in the form of a stick of cellulose-based material carrying the reagent at one end.

3.    A diagnostic test probe as claimed in claim 2, wherein the stick is a stick of rolled paper.

4.    A diagnostic test probe as claimed in claim 1, in the form of a rod of porous synthetic plastics material carrying the reagent at one end.

5.    A diagnostic test probe as claimed in any one of claims 1 to 4, wherein the reagent is chosen to react with the enzyme suspected to be in the sample so as to generate a colour change.

6.    A diagnostic test probe as claimed in any one of claims 1 to 5, wherein the probe is self-supporting and substantially rigid in use.

7.    A diagnostic test probe as claimed in any one of claims 1 to 6, wherein the tip has a diameter of from 0.5 mm to 10 mm.

8.    A method of testing for the presence in a sample of an enzyme by the use of a diagnostic test probe as claimed in any one of claims 1 to 7, which method comprises applying the reagent-carrying tip of the probe to the sample, permitting reaction to take place between the reagent and the enzyme, and observing a signal indicating the presence in the sample of the enzyme.

9.  A method as claimed in claim 8, wherein the sample is a bacterial colony grown on a nutrient gel.

10. A method of typing bacteria, which method comprises growing a colony of the bacteria to be typed on a nutrient gel, providing a diagnostic test probe comprising a handle and a tip which carries a reagent to react with an enzyme and thereby generate a colour signal, applying the reagent-carrying tip of the probe to the bacterial colony, permitting reaction to take place between the reagent and the enzyme, observing any resulting colour change and using the information to type the bacteria.

10

12

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | DE-A-2 136 880 (B.J.RADOLA) *The whole document* | 1-8 | C 12 Q 1/00<br>C 12 Q 1/04 |
| Y | EP-A-0 016 962 (BOEHRINGER MANNHEIM) *Page 7, line 19 - page 8, line 4; figures 1,2; claims 8-11* | 1-8 | |
| Y | US-A-3 891 507 (C.B.BREUER) *The whole document* | 1-8 | |
| Y | US-A-3 547 780 (F.A.FINNERTY et al.) *The whole document* | 1-8 | |
| Y,A | US-A-3 122 480 (F.TUMER & G.WATSON) *The whole document* | 1-10 | **TECHNICAL FIELDS SEARCHED (Int. Cl. ³)** |
| Y | US-A-3 691 018 (T.McNUMARA & R.WINER) *The whole document* | 1-8 | C 12 Q<br>G 01 N |
| Y | US-A-3 926 732 (E.ROSEN & H.ROSEN) *The whole document* | 1-8 | |

The present search report has been drawn up for all claims

| Place of search | Date of completion of the search | Examiner |
|---|---|---|
| THE HAGUE | 21-03-1984 | OSBORNE H.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82